# EUROPEAN PATENT APPLICATION

(11) **EP 4 545 031 A1**
(43) Date of publication of application: **30.04.2025**
(21) Application number: 23826578.9
(22) Date of filing: 24.06.2023
(51) Int. Cl.: A61B 18/14

(54) **MEDICAL ABLATION CATHETER, ABLATION SYSTEM, AND ABLATION METHOD**

(30) Priority: 24.06.2022 CN 202210730368
(71) Applicant: Shanghai Golden Leaf Med Tec Co., Ltd, Xuhui District Shanghai 200231 (CN)
(72) Inventor: CAO, Hongguang, Shanghai 200231 (CN); CHEN, Haidong, Shanghai 200231 (CN); JI, Liang, Shanghai 200231 (CN); SHEN, Meijun, Shanghai 200231 (CN)
(74) Representative: Wang, Bo
(86) International application number: PCT/CN2023/102013
(87) International publication number: WO 2023/246933

(57) **Abstract**

A medical ablation catheter, an ablation system, and an ablation method. The medical ablation catheter comprises a first catheter (1), located at the distal end of the medical ablation catheter; a second catheter (2), comprising an inner catheter (21) and an outer catheter (22), the outer catheter (22) being sleeved on the outer side of the inner catheter (21) and being capable of sliding with respect to the inner catheter (21); a conductive part (3), connected to the first catheter (1) and the inner catheter (21), the distal end of the conductive part (3) being fixed within the first catheter (1), the proximal end of the conductive part (3) being fixed on the inner catheter (21); an adjustment part (4), the distal end thereof being fixed within the first catheter (1), the proximal end thereof being movably arranged within the inner catheter (21) in a penetrating manner. The conductive part (3) can be switched between a linear state and an expanded state, and ablation is performed when the conductive part (3) is in the expanded state. The medical ablation catheter can implement various ablation modes and different operational forms can be changed according to requirements, so as to improve applicability, and expandable electrodes (5, 32) are used to match areas to be ablated, so as to improve adaptability.

## Description

### BACKGROUND

### Technical Field

The present invention relates to the technical field of medical devices, relates to a medical ablation catheter, and relates to an ablation system including the medical ablation catheter and an ablation method based on the medical ablation catheter.

### Related Art

Since first implementation in 1969, the ablation procedure for heart diseases has undergone a lot of innovation and rapid development. The ablation procedure was first configured to treat a patient with supraventricular tachycardia accompanied with an accessory pathway and a pre-excitation syndrome. Nowadays, the ablation procedure is usually configured to treat atrial flutter, atrial fibrillation, and ventricular arrhythmia.

A purpose of the ablation procedure is to destroy potential arrhythmic tissue and create a transmural and continuous permanent lesion. In the prior art, percutaneous catheter ablation achieving pulmonary vein isolation in atrial tissue by radio-frequency ablation (RFA for short) and cryotherapy has become a widely accepted surgical method for treating atrial fibrillation. Currently, there is also a surgical method for treating atrial fibrillation by atrial appendage ablation. In the ablation procedure, radio frequency is the most commonly used energy supply manner currently, and other available energy supply manners further include microwave, high-intensity focused ultrasound, low-intensity collimated ultrasound, laser, cryogenic energy, heated saline, and the like.

Irreversible electroporation (IRE for short) is a new technique in the field of tumor ablation. The IRE uses high-voltage narrow pulses to act on a lesion location, to cause nanoscale permanent perforation of a cell membrane, thereby resulting in tumor cell apoptosis. Compared with the RFA, the IRE may produce an ablation lesion without consequences of heat conduction, that is, can preserve a surrounding tissue structure. A corresponding voltage pulse is usually referred to as pulsed field ablation (PFA for short). For the RFA and the PFA, many technical problems that need to be urgently resolved still exist, such as how to improve ablation efficiency and ablation safety to achieve a purpose of quick, safe, and effective treatment of heart diseases such as arrhythmia.

### SUMMARY

A primary technical problem to be resolved in the present invention is to provide a medical ablation catheter improving an ablation effect.

Another technical problem to be resolved in the present invention is to provide an ablation system including the above medical ablation catheter.

Still another technical problem to be resolved in the present invention is to provide an ablation method based on the above medical ablation catheter.

To achieve the above purposes, the present invention adopts the following technical solutions:
According to a first aspect of embodiments of the present invention, a medical ablation catheter is provided, including:
a first catheter, located on a distal end of the medical ablation catheter;
a second catheter, including an inner catheter and an outer catheter, where the outer catheter is sleeved on an outer side of the inner catheter, and is slidable relative to the inner catheter;
a conductive portion, configured to connect to the first catheter and the inner catheter, where a distal end of the conductive portion is fixed in the first catheter, and a proximal end of the conductive portion is fixed on the inner catheter; and
an adjustment portion, where a distal end of the adjustment portion is fixed in the first catheter, and a proximal end of the adjustment portion is arranged to movably extend through the inner catheter.

The conductive portion may be switchable between a linear state and an expanded state, and ablation is performed when the conductive portion is in the expanded state.

Preferably, the medical ablation catheter further includes a first expandable electrode.

The first expandable electrode is located on a distal end of the first catheter, and the first expandable electrode is made of a preformed metal wire or made by carving a metal tube.

Preferably, the conductive portion includes a plurality of second expandable electrodes configured to perform ablation, and each of the second expandable electrodes is made of a preformed flexible metal wire.

Preferably, the conductive portion further includes a plurality of elastic tubes and a support wire.

The second expandable electrode is arranged between two adjacent elastic tubes, and two ends of the second expandable electrode each have a tail portion configured to connect to each of the elastic tubes.

Preferably, the conductive portion further includes a preformed support wire.

The support wire is made of a shape memory alloy, and is arranged to extend through the plurality of elastic tubes and the plurality of second expandable electrodes.

Preferably, the first expandable electrode and/or the second expandable electrode is provided with a pressure sensor.

The pressure sensor is configured to display an adherence position of the first expandable electrode based on a detected pressure, or the pressure sensor is configured to display adherence positions and/or an adherence quantity of the second expandable electrodes based on the detected pressure.

Preferably, the first catheter is provided with a first controllably bendable section, to control a shape of a to-be-ablated region of the first expandable electrode.

Preferably, a surface of the first catheter is provided with a plurality of positioning scanning apparatuses, to transmit scanning data to an external device, to form a three-dimensional (3D) image of the to-be-ablated region.

Preferably, the medical ablation catheter further includes a cold saline perfusion tube. The cold saline perfusion tube is arranged to extend through the first catheter, a first end of the cold saline perfusion tube is located in the first expandable electrode, and a second end of the cold saline perfusion tube is in communication with a cold saline supply apparatus, to deliver cold saline when a surface temperature of the first expandable electrode reaches a preset value.

Preferably, the adjustment portion is arranged to extend through and is movable relatively the inner catheter, and the conductive portion is fixedly connected to the inner catheter and is fixedly connected to the first catheter.

Preferably, the metal wire of the second expandable electrode is finer than the metal wire of the first expandable electrode.

According to a second aspect of the embodiments of the present invention, an ablation system is provided, including the above medical ablation catheter and an ablation energy source. The ablation energy source is configured to provide energy to the ablation catheter.

Preferably, the ablation energy source includes at least an energy source for pulsed field ablation (PFA) and an energy source for radio-frequency ablation (RFA).

According to a third aspect of the embodiments of the present invention, an ablation method based on the medical ablation catheter is provided, including the following steps:
delivering the first catheter and the conductive portion by using the second catheter;
pushing the first catheter, so that the first catheter and the conductive portion extend out toward a distal end from the second catheter;
pulling and rotating the adjustment portion, to cause the second expandable electrode of the conductive portion to be adjusted to the expanded state, or to cause the first expandable electrode of the first catheter to be adjusted to the expanded state;
selectively energizing a quantity of electrodes on the conductive portion that need to be energized;
stopping energization;
withdrawing the adjustment portion, to receive the first catheter and the conductive portion within the second catheter; and
withdrawing the second catheter.

Preferably, the ablation method further includes the following steps:
pulling and rotating the adjustment portion after the energization is stopped, to cause the conductive portion to be adjusted to the linear state and be withdrawn into the second catheter;
causing the first expandable electrode on the first catheter to be energized; and
stopping the energization, and withdrawing the first catheter into the second catheter.

Compared with the prior art, according to the medical ablation catheter provided in the present invention, stretching length of the conductive portion may be adjusted by stretching the proximal end of the adjustment portion, and a rotation angle of the conductive portion is adjusted by rotating the proximal end of the adjustment portion, thereby jointly adjusting an operating form of the conductive portion. During specific use, the conductive portion may be adjusted to different shapes such as a circular ring, a spiral, and a straight line as required. When the conductive portion is in the shape of the spiral, magnitudes of a pitch and an outer diameter may be adjusted by stretching or rotating the adjustment portion. Therefore, a plurality of ablation manners can be achieved by using the medical ablation catheter, and different operating forms may be changed as required, thereby improving applicability and convenience in use of the medical ablation catheter.

### BRIEF DESCRIPTION OF THE DRAWINGS

FIG. 1A is a schematic structural diagram of a medical ablation catheter according to a first embodiment of the present invention.
FIG. 1B is a schematic structural diagram of a second expandable electrode in FIG. 1.
FIG. 1C is another schematic structural diagram of a first expandable electrode in FIG. 1.
FIG. 2 is a schematic structural diagram of another usage state of a medical ablation catheter according to a first embodiment of the present invention.
FIG. 3 is a partial enlarged view of a portion X in FIG. 2.
FIG. 4 is a schematic structural diagram of a medical ablation catheter according to a second embodiment of the present invention.
FIG. 5 is a schematic structural diagram of a carved first expandable electrode according to a third embodiment of the present invention.
FIG. 6 is a schematic structural diagram of another carved first expandable electrode according to a third embodiment of the present invention.
FIG. 7 is a schematic structural diagram of a woven ball-shaped support according to a third embodiment of the present invention.
FIG. 8 is a schematic structural diagram of another woven ball-shaped support according to a third embodiment of the present invention.
FIG. 9 is a flowchart of an ablation method according to a sixth embodiment of the present invention.
FIG. 10A is a schematic structural diagram of a left atrial appendage without thrombus being formed therein.
FIG. 10B is a partial enlarged view of a circled region in FIG. 10A in which a thrombus has been formed in a left atrial appendage.

### DETAILED DESCRIPTION

Technical content of the present invention is described in detail below with reference to drawings and specific embodiments.

According to a medical ablation catheter provided in the embodiments of the present invention, intensive ablation is achieved through a first expandable electrode, and extensive ablation is achieved through a spiral structure formed by a plurality of second expandable electrodes, thereby taking precision of ablation into account while ensuring ablation efficiency, to improve an overall ablation effect. In addition, according to the medical ablation catheter provided in the embodiments of the present invention, a relatively large elastic force is provided by using the spiral structure, to promote adherence of the second expandable electrodes, and a metal wire of each second expandable electrode can be caused to vary with a shape of a blood vessel or thrombus through flexibility of the fine metal wire of the expandable electrode, to provide a relatively small force, and promote the adherence of the second expandable electrodes. It may be learned from the above that the medical ablation catheter provided in the embodiments of the present invention has a two-stage elastic structure (the spiral structure and a spherical structure), to ensure desirable contact with a to-be-ablated region, and improve conformability of the medical ablation catheter.

The medical ablation catheter and an ablation method thereof provided in the present invention are described in detail below with reference to the embodiments.

### <First embodiment>

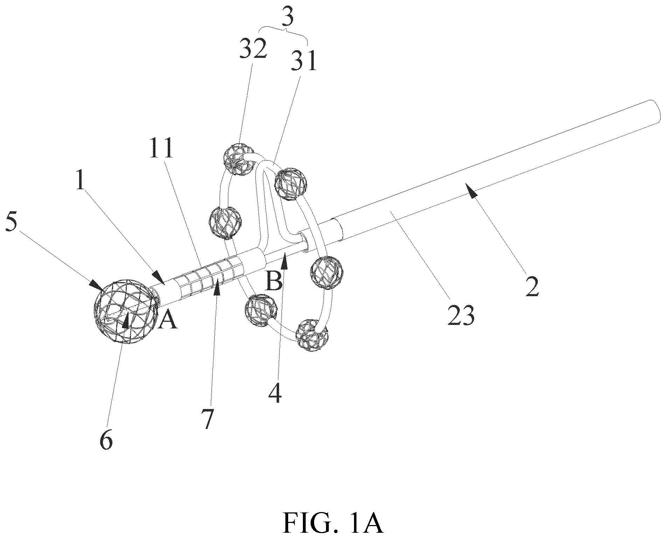

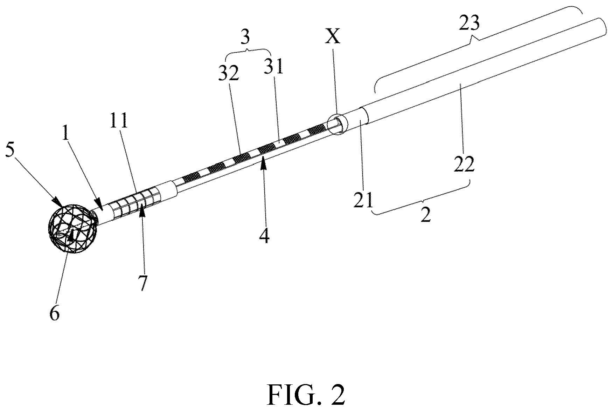

As shown in FIG. 1A and FIG. 2, a medical ablation catheter provided in an embodiment of the present invention includes a first catheter 1, a second catheter 2, a conductive portion 3, an adjustment portion 4, a first expandable electrode 5, and a plurality of second expandable electrodes 32. The first expandable electrode 5 and the plurality of second expandable electrodes 32 are preferably spherical electrodes, but are not limited to, spherical electrodes. The first expandable electrode or each of the second expandable electrodes is made of a preformed metal wire (including by weaving, welding, carving, and the like), or made by carving a metal tube. Either the first expandable electrode or the second expandable electrode may be switched between an expanded state and a linear state.

As shown in FIG. 1A and FIG. 2, a distal end of the first catheter 1 (that is, an end A in FIG. 1A) is provided with the first expandable electrode 5. The second catheter 2 includes an inner catheter 21 and an outer catheter 22. The inner catheter 21 is located on a proximal end of the first catheter 1 (that is, an end B in FIG. 1A), and is arranged spaced apart from the first catheter 1. The outer catheter 22 is a multi-lumen catheter, and includes at least two lumens. The outer catheter 22 is sleeved on an outer side of the inner catheter 21, and is slidable relative to the inner catheter 21. A distal end of the conductive portion 3 is fixed to the proximal end of the first catheter 1, and a proximal end of the conductive portion 3 is fixed to the inner catheter 21. The adjustment portion 4 is slidable relative to and arranged to extend through a lumen of the inner catheter 21, and is connected to a control handle (not shown). A distal end of the adjustment portion 4 is fixedly connected to the first catheter 1. Therefore, the first catheter 1 may be controlled to move forward or backward through extension or withdrawal of the adjustment portion 4, so that the first catheter 1 moves away from or close to the inner catheter 21. In addition, the first catheter 1 and the conductive portion 3 can be driven to rotate by rotating the adjustment portion 4. It may be learned that an operating form of the conductive portion 3 can be adjusted by using the adjustment portion 4, so that the conductive portion 3 is switched between the linear state and the expanded state.

In this embodiment, during specific use, a position on which ablation needs to be performed (for example, a left atrial appendage, or a pulmonary vein ostium) is determined based on scanning and mapping results. If extensive ablation is required, the adjustment portion 4 is pulled, so that the conductive portion 3 is distributed in a shape of a circular ring (that is, the expanded state shown in FIG. 1A), and is switched to the expanded state, to perform the extensive ablation through the plurality of second expandable electrodes 32 (a spiral or annular region formed by the plurality of second expandable electrodes 32 together). Alternatively, when intensive ablation is required, the entire conductive portion 3 may be switched to the linear state. The conductive portion 3 is in a shape of a straight line (that is, the linear state shown in FIG. 2), to perform the intensive ablation through the first expandable electrode 5 (only a region with which a spherical surface of the first expandable electrode 5 is in contact).

Therefore, an area of an ablation region in the intensive ablation is less than that in the above extensive ablation. Therefore, a plurality of ablation manners can be achieved by using the medical ablation catheter, and different operating forms may be changed as required, so that precision of ablation can be taken into account while ensuring ablation efficiency, thereby improving an adherence capability, conformability, and convenience in use of the medical ablation catheter.

In the expanded state, the conductive portion 3 can change a pitch and an outer diameter based on a rotation degree and a stretching degree of the adjustment portion 4. For example, if the adjustment portion 4 is rotated less than one circle, the conductive portion 3 is in a shape of an arc. If the adjustment portion 4 is rotated one circle, the conductive portion 3 is in a shape of the circular ring. If the adjustment portion 4 is rotated more than one circle, the ring shape becomes a spiral shape, and more rotation circles indicate a smaller outer diameter of the spiral shape. In addition, the pitch of the spiral shape may be adjusted by stretching the adjustment portion 4. The first catheter 1 closer to the second catheter 2 indicates the smaller pitch of the spiral shape. The first catheter 1 farther away from the second catheter 2 indicates the larger pitch of the spiral shape. Specifically, adaptive adjustment may be performed based on an actual ablation requirement, thereby improving convenience of ablation.

It may be understood that when the conductive portion 3 is in the expanded state to perform the extensive ablation, the shape of the conductive portion 3 is quickly changed by rotating the adjustment portion 4, so that the second expandable electrode 32 can quickly approach and be adhered to a to-be-ablated region, to achieve initial adjustment of an adherence force. In this way, relative positions between the plurality of second expandable electrodes 32 is deformed with the to-be-ablated region, to conform to the to-be-ablated region. Further, the relatively flexible metal wire of the second expandable electrode 32 is tightly adhered to the to-be-ablated region, to deform each second expandable electrode 32, so as to achieve precise adjustment of the adherence force. In this way, each metal wire of each second expandable electrode 32 conforms to a region in contact with the metal wire. Therefore, in this embodiment, the adherence force between the second expandable electrode 32 and the to-be-ablated region can be more precisely controlled in a two-stage adjustment manner, to improve an ablation effect. In addition, in this embodiment, the metal wire forming the second expandable electrode 32 is finer and/or more flexible than the metal wire forming the first expandable electrode 5, so that the second expandable electrode more easily conforms to the to-be-ablated region in an extensive ablation state.

As shown in FIG. 1A, in the above embodiment, preferably, a middle part of the first catheter 1 is a first controllably bendable section 11, to control a shape of an intensive to-be-ablated region of the first expandable electrode 5. Specifically, in this embodiment, the first controllably bendable section 11 may be a snake bone tube, or a plurality of metal wires (not shown in the figure) may be connected to the first controllably bendable section 11. Each of the metal wires extends through the first catheter 1 and the second catheter 2 and protrudes from the second catheter 2, to control a bending degree of the first controllably bendable section 11 by pulling different metal wires. When the medical ablation catheter is in an intensive ablation mode, an operator may adjust the bending degree of the first controllably bendable section 11 by pulling different metal wires, thereby adjusting a specific position of the first expandable electrode 5 in the to-be-ablated region. Therefore, a position of the second expandable electrode 32 can be freely controlled by using combination of the first controllably bendable section 11 and the plurality pf metal wires, to achieve precise ablation, and improve the overall ablation effect.

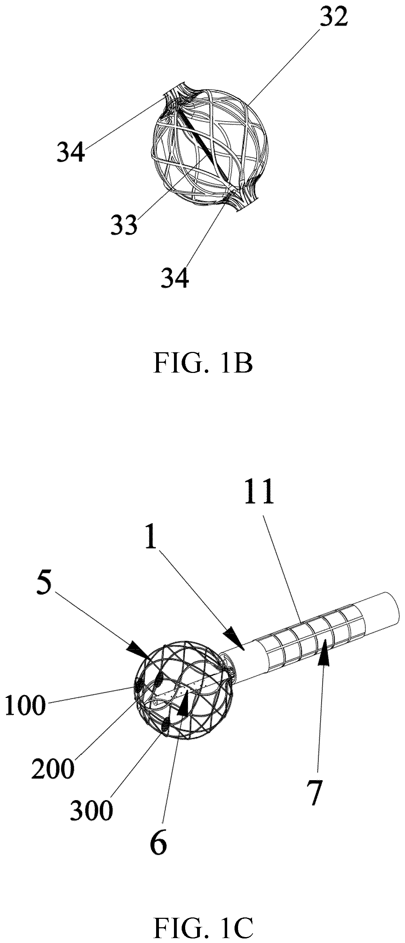

As shown in FIG. 1A and FIG. 1B, in the above embodiment, the conductive portion 3 includes a plurality of elastic tubes 31, the plurality of second expandable electrodes 32, and a support wire 33. Specifically, in this embodiment, the elastic tubes 31 are arranged at intervals in sequence, and include two elastic tubes 31A on end portions and five elastic tubes 31B at a middle part. The elastic tubes 31A on the two ends each are substantially bent into a shape of Z, and are respectively fixed on the first catheter 1 and the second catheter 2. The remaining five elastic tubes 31B each are in a shape of an arc shape. A mounting gap is formed between two adjacent elastic tubes 31, to be configured to mount the second expandable electrode 32. Two ends of each second expandable electrode 32 each have a tail portion configured to be connected to the elastic tube 31. The tail portion is arranged to extend through the elastic tube 31, and the second expandable electrode 32 is fixed between the two adjacent elastic tubes 31 by adhesive dispensing. In addition, after the plurality of elastic tubes 31 are fixed to the plurality of second expandable electrodes 32, the plurality of elastic tubes 31 and the plurality of second expandable electrodes 32 are stretched in a straight line, and the support wires 33 is arranged to extend through the plurality of elastic tubes 31 and plurality of second expandable electrodes 32 in sequence. The support wire 33 is made of a shape memory alloy, and has a shape memory function. The support wire 33 is preformed, so that each of the elastic tubes 31 and the second expandable electrode 32 may be driven by using the support wire 33 to perform adaptive elastic deformation (in the expanded state), to meet different ablation requirements.

Referring to FIG. 1 and FIG. 2, in the above embodiment, preferably, the second expandable electrode 32 may elastically extend and retract, to expand into a mesh like sphere in a state in which the ablation catheter requires the extensive ablation (referring to the state shown in FIG. 1), and retract into a shape of a straight line in a state in which the ablation catheter requires the intensive ablation (referring to the state shown in FIG. 2). Specifically, in this embodiment, the second expandable electrode 32 is made by using a spherical jig through a hot forming process. A specific process is as follows: In a first step, a nickel titanium tube with a preset diameter (preferably, a diameter is less than 1 mm) is selected. In a second step, an outer wall of the nickel titanium tube is carved (for example, the outer wall of the nickel titanium tube is carved into a grid shape to form a metal wire with a thickness in a range of 0.08-0.1 mm), thereby forming a grid-shaped nickel titanium wire tube. In a third step, the spherical jig with a preset size is arranged to extend through the grid-shaped nickel titanium tube. In a fourth step, high-temperature setting is performed at a temperature in a range of 300-400°C, to form a spherical support. In a fifth step, the set spherical support is cooled, and an electrode is bonded or welded to the spherical support, to obtain an expandable electrode that can switch between a retracted state and an expanded state. Finally, the elastic tube 31 and the second expandable electrode 32 are inserted into the support wire 33, and finally the support wire 33 is fixed onto the first catheter 1. The metal wire of the second expandable electrode 32 is finer than the metal wire of the first expandable electrode 5, to be more tightly adhered to the to-be-ablated region.

Therefore, the shape of the second expandable electrode 32 varies with a position of the second catheter 2. When the extensive ablation needs to be performed, the second expandable electrode 32 correspondingly expands into the grid-shaped spherical structure, to ensure a maximum ablation area, and conform to the to-be-ablated region. When the intensive ablation needs to be performed, the second expandable electrode 32 becomes a straight line, to avoid interference with the ablation of the first expandable electrode 5, and the second expandable electrode can enter inner of a blood vessel more smoothly, to further improve the usage convenience of the ablation catheter.

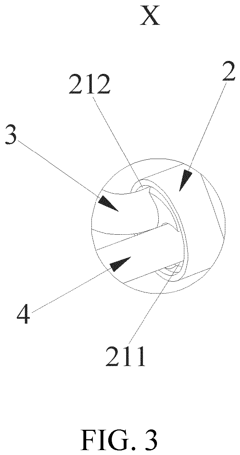

As shown in FIG. 3, in the above embodiment, the inner catheter 21 is provided with a first channel 211 and a second channel 212. The adjustment portion 4 is arranged to extend through the first channel 211 and may move relative to the inner catheter 21. A fixed end 34 of the conductive portion 3 is fixed to the inner catheter 21, and the support wire 33 is arranged to extend through the second channel 212. Therefore, the conductive portion 3 can be completely separated from the adjustment portion 4 by using the first channel 211 and the second channel 212, to avoid interference between the two portions, thereby improving stability of the conductive portion 3 and flexibility of the adjustment portion 4. In other embodiments, an effect of reducing sliding friction may be achieved by adding a coating on a surface of the adjustment portion 4. In addition, shapes of the first channel 211 and the second channel 212 may be the same or different. For example, the first channel 211 and the second channel 212 are both cylindrical, or one is cylindrical and the other is strip-shaped. Therefore, adaptive adjustment may be made based on specific shapes of the conductive portion 3 and the adjustment portion 4.

In addition, in the above embodiment, preferably, a distal end of the outer catheter 22 is a second controllably bendable section 23, to control an ablation direction of the medical ablation catheter. In this embodiment, structures of the second controllably bendable section 23 and the first controllably bendable section 11 may be the same or different, as long as a controllable bending effect can be achieved. When cardiac ablation is performed by using the medical ablation catheter, not only can the overall ablation direction of the medical ablation catheter (that is, an overall direction of entering a heart) be controlled through the second controllably bendable section 23 to arrive at a to-be-ablated region inside the heart, but also a shape of the to-be-ablated region of the first expandable electrode 5 can be controlled by the first controllably bendable section 11, thereby improving control precision of the shape of the to-be-ablated region, or a shape of the to-be-ablated region of the second expandable electrode 32 can be changed by using the adjustment portion 4. More preferably, the second controllably bendable section 23 is further provided with a development ring, to display a position of the second controllably bendable section 23.

In the above embodiment, preferably, the plurality of second expandable electrodes 32 are independently controlled. Specifically, in FIG. 1, a total of six second expandable electrodes 32 are arranged, which are now numbered A, B, C, D, E, and F. During the specific extensive ablation, a final ablated region may be determined based on three-dimensional (3D) scanning and mapping results and an actual situation of a patient. In addition, in the ablated region, regions being heavy and intensive ablation regions, and regions requiring merely slight ablation may be determined. In general, the three second expandable electrodes 32 A, C, and E may be set to be positively charged, and the three second expandable electrodes 32 B, D, and F may be negatively charged, to achieve uniform ablation. Alternatively, the three second expandable electrodes 32 A, B, and C may be set to be positively charged, and the three second expandable electrodes 32 D, E, and F may be set to be negatively charged, to form a corresponding magnetic field. Alternatively, during the ablation, if the scanning and mapping results indicates that regions where the second expandable electrodes 32 E and F are located does not require ablation, merely the four second expandable electrodes 32 A, B, C, and D may be used for the ablation. In addition, a specific manner of energizing is mapped based on the scanning result. Therefore, the plurality of second expandable electrodes 32 are independently controller, so that corresponding ablation operations can be performed based on different usage conditions, thereby improving operability of the ablation process.

As shown in FIG. 1, in the above embodiment, preferably, a surface of the first controllably bendable section 11 has a plurality of block-shaped positioning scanning apparatuses 7. Scanning data can be transmitted to an external device through each of the block-shaped positioning scanning apparatuses 7, to form a 3D image of the to-be-ablated region, thereby achieving a visually minimally invasive surgical operation, and providing convenience for a doctor.

As shown in FIG. 1C, optionally, in this embodiment, the first expandable electrode 5 is further provided with a temperature sensor 100 and a pressure sensor 200. The temperature sensor is configured to detect an ablation temperature, to control a temperature range during the ablation, thereby avoiding a situation of excessive ablation or insufficient ablation. The pressure sensor is configured to detect a pressure between the second expandable electrode 32 and the to-be-ablated region, to determine whether the second expandable electrode 32 at the position is in full contact with the to-be-ablated region. If the second expandable electrode 32 at the position is not in full contact with the to-be-ablated region, the shape of the to-be-ablated region may be readjusted or the second expandable electrode 32 at the position may be controlled to be de-energized, and ablation is performed on another position, to avoid energy waste and improve economic benefit. It may be understood that in an extensive ablation state, when the second expandable electrode 32 comes into contact with the to-be-ablated region, adaptive deformation may be performed based on a shape of an inner wall of the to-be-ablated region, so as to perfectly fit with the inner wall of the to-be-ablated region, thereby improving the ablation effect on the to-be-ablated region, saving ablation time, and improving convenience and conformability in use of the medical ablation catheter. Optionally, the first expandable electrode 5 is further provided with a potential detector 300, to control continuation or cessation of the ablation based on presence or absence of a potential.

**In** the above embodiment, preferably, the first expandable electrode 5 is a reticulated spherical electrode, a size of the first expandable electrode 5 is greater than or equal to a size of the second expandable electrode 32, but specific sizes of the first expandable electrode and the second expandable electrode are not limited in the present invention. During specific use, energized states of the second expandable electrode 32 and the first expandable electrode 5 may be controlled as required. For example, when the second expandable electrode 32 is energized and the first expandable electrode 5 is de-energized, the conductive portion 3 may be adjusted to be in the shape of the circular ring (the expanded state as shown in FIG. 1) through the adjustment portion 4, and finally, the extensive ablation is achieved through pulsed field ablation (PFA). When the second expandable electrode 32 is de-energized and the first expandable electrode 5 is energized, the conductive portion 3 may be adjusted to be in the shape of the straight line (the linear state as shown in FIG. 2) through the adjustment portion 4, to achieve the intensive ablation by using radio frequency (RF) energy. Therefore, different radio-frequency ablation (RFA) modes may be selected based on different usage requirements, to improve the applicability of the medical ablation catheter.

### <Second embodiment>

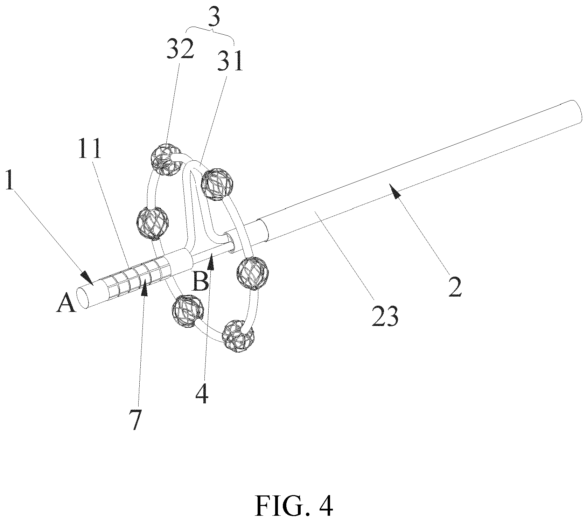

FIG. 4 shows a medical ablation catheter according to a second embodiment of the present invention. Compared with the first embodiment, a difference is that the medical ablation catheter omits a first expandable electrode 5 and includes merely second expandable electrodes 32.

It may be understood that, since the first expandable electrode 5 is omitted in this embodiment, the medical ablation catheter can perform extensive ablation through merely a plurality of second expandable electrodes 32, and cannot perform intensive ablation.

Other than the above difference, other structures in this embodiment are the same as those in the first embodiment, and details are not described again.

### <Third embodiment>

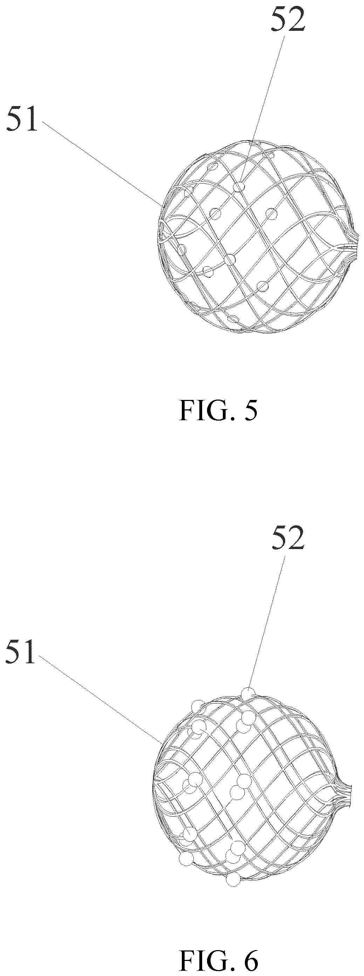

FIG. 5 to FIG. 8 are schematic structural diagrams of a first expandable electrode 5 of a medical ablation catheter according to a third embodiment of the present invention. A difference between this embodiment and the first embodiment is that the medical ablation catheter omits a plurality of second expandable electrode 32 and includes merely the first expandable electrode 5.

It may be understood that, since the plurality of second expandable electrode 32 are omitted in this embodiment, the medical ablation catheter can perform intensive ablation through merely the first expandable electrode 5, and cannot perform extensive ablation. In addition, the first expandable electrode 5 may be implemented as a large support insulated as a whole with exposed metal at some positions serving as electrodes, or may be implemented as a large insulated support with metal electrodes mounted at some positions.

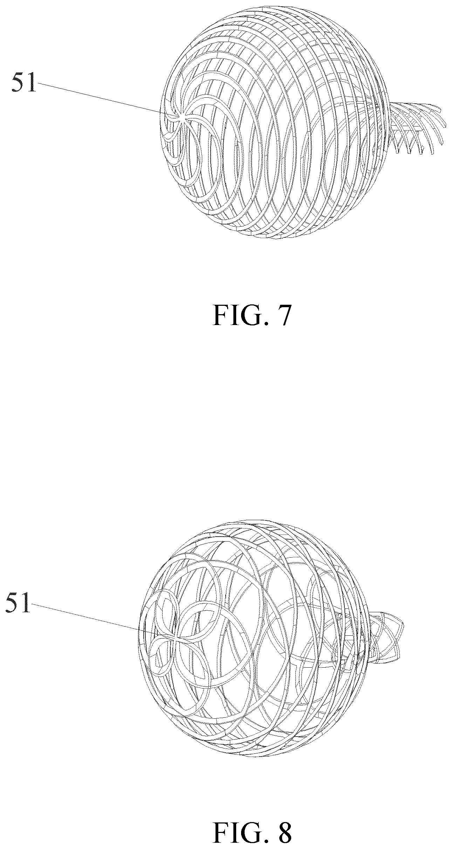

Specifically, FIG. 5 to FIG. 8 show four structural forms of the first expandable electrode 5. As shown in FIG. 5 and FIG. 6, the first expandable electrode 5 includes a ball-shaped support 51 and an electrode piece 52. The ball-shaped support 51 is formed by carving, and the electrode piece 52 in FIG. 5 is adhered to the ball-shaped support 51 by adhesive dispensing, to form the first expandable electrode 5. The electrode piece 52 in FIG. 6 is fixed to the ball-shaped support 51 by welding, to form the first expandable electrode 5. FIG. 7 and FIG. 8 each show another structural form of the ball-shaped support 51. The ball-shaped support 51 in FIG. 7 is formed by unidirectional weaving of metal wires. In other words, all of the metal wires are woven clockwise (or may be woven counterclockwise). The ball-shaped support 51 in FIG. 8 is formed by bidirectional weaving of metal wires. In other words, some of the metal wires are woven clockwise, and the other some of the metal wires are woven counterclockwise. Therefore, the ball-shaped support 51 formed in the above carving or weaving manner has desirable elasticity, so that the electrode piece 52 can more easily conform to a to-be-ablated region, thereby improving an ablation effect.

It may be understood that the above specific structural forms of the first expandable electrode 5 are only preferred implementations, and does not constitute a limitation on a shape of the first expandable electrode 5. In other embodiments, the first expandable electrode 5 may be adaptively deformed as required, to satisfy different ablation requirements.

Other than the above difference, other structures in this embodiment are the same as those in the first embodiment, and details are not described again.

### <Fourth embodiment>

Based on the first embodiment, preferably, the medical ablation catheter further includes a control handle (not shown in the figure).

Specifically, both the adjustment portion 4 and a metal guide wire are connected to the control handle, to control an operating form of the conductive portion 3 and bending degrees of the first controllably bendable section 11 and the second controllably bendable section 23 through the control handle. Specifically, when the control handle is configured to pull the adjustment portion 4, shapes of the plurality of second expandable electrodes 32 may be adjusted, to vary between different working forms, and form different ablation modes. When the metal guide wire is pulled by using the control handle, the bending degree of the first controllably bendable section 11 may be controlled, thereby controlling the specific position of each of the second expandable electrodes 32.

It may be understood that, in general, the extensive ablation mode and the intensive ablation mode are not active at a same time. Therefore, when the medical ablation catheter is in the extensive ablation mode, the control handle does not control the metal guide wire. In addition, when the medical ablation catheter is in the intensive ablation mode, the control handle does not control the adjustment portion 4, to avoid mutual impact.

### <Fifth embodiment>

As shown in FIG. 1, based on the first embodiment, preferably, the medical ablation catheter further includes a cold saline perfusion tube 6. The cold saline perfusion tube 6 is arranged to extend through the first catheter 1, a first end of the cold saline perfusion tube 6 is located in the first expandable electrode 5, and a second end of the cold saline perfusion tube 6 is in communication with a cold saline supply apparatus (not shown in the figure), to deliver cold saline when a surface temperature of the first expandable electrode 5 reaches a preset value, thereby reducing the surface temperature of the first expandable electrode 5.

During specific operation, the surface temperature of the first expandable electrode 5 may be preset to a maximum of 50°C. When the temperature sensor senses that the surface temperature of the first expandable electrode 5 reaches the preset value (that is, 50°C), the temperature sensor sends a signal to the cold saline supply apparatus. The cold saline supply apparatus receives the signal and delivers the cold saline through the cold saline perfusion tube 6, to reduce the surface temperature of the first expandable electrode 5. The cold saline is physiological saline, which is absorbed by a human body without any negative effect after being injected into the human body to cool the first expandable electrode 5. Therefore, through monitoring of the surface temperature of the first expandable electrode 5 in real time, excessive ablation of the to-be-ablated region as a result of an excessively high temperature can be avoided.

### <Sixth embodiment>

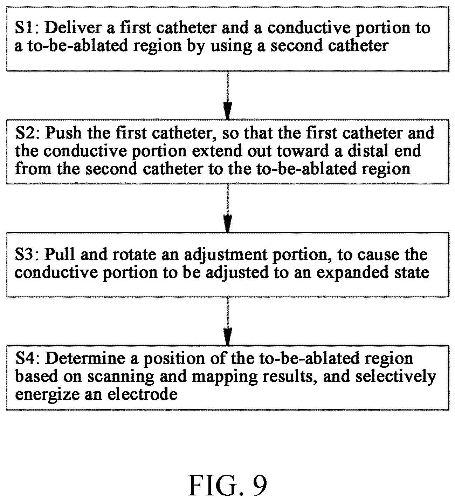

FIG. 9 shows an ablation method based on the medical ablation catheter according to a sixth embodiment of the present invention. The method includes the following steps:
S1: Deliver a first catheter and a conductive portion to a to-be-ablated region by using a second catheter.
S2: Push the first catheter, so that the first catheter and the conductive portion extend out toward a distal end from the second catheter to the to-be-ablated region.
S3: Pull and rotate an adjustment portion, to cause the conductive portion to be adjusted to an expanded state.
   A second end of the adjustment portion 4 is pulled and rotated, to cause the first catheter 1 to be close to the inner catheter 21 and to rotate relative to the inner catheter 21, thereby adjusting the conductive portion 3 to the suitable expanded state;
S4: Determine a position of the to-be-ablated region based on scanning and mapping results, and selectively energize an electrode.

This step includes the following two situations, one of the two steps needs to be selected based on an actual situation of the to-be-ablated region, or two steps are performed in sequence:
S41: Determine a position of the to-be-ablated region based on scanning and mapping results, and selectively energize the second expandable electrodes of a corresponding quantity and corresponding positions.

Different quantities of the second expandable electrodes (including the quantity of selected second expandable electrodes 32 or a quantity of electrode pieces 52 on each of the second expandable electrodes) are selectively energized, so that different ablation capabilities can be obtained. A position and a size of the to-be-ablated region is determined based on the scanning and mapping results, and the second expandable electrode 32 of the corresponding quantity and the corresponding positions needs to be selectively energized, so that the second expandable electrode 32 or the electrode piece 52 located in the to-be-ablated region is energized to perform ablation.

It may be understood that this step may be repeatedly performed until the ablation is completed.

S42: Selectively energize a first expandable electrode based on an ablation situation, to perform the ablation again or end the ablation.

If intensive ablation needs to be performed by using the first expandable electrode 5 during the ablation, the conductive portion 3 is adjusted to a linear state through the adjustment portion 4 and is withdrawn into the outer catheter 2, and then the first expandable electrode 5 is energized, to achieve the intensive ablation by using the RF energy until the entire ablation process is completed.

It may be understood that the above steps may be adaptively adjusted as required. When a specific ablation mode is needed, the medical ablation catheter can be adjusted to the corresponding shape, so that different ablation modes may be selected based on different to-be-ablated regions, thereby improving convenience of the ablation.

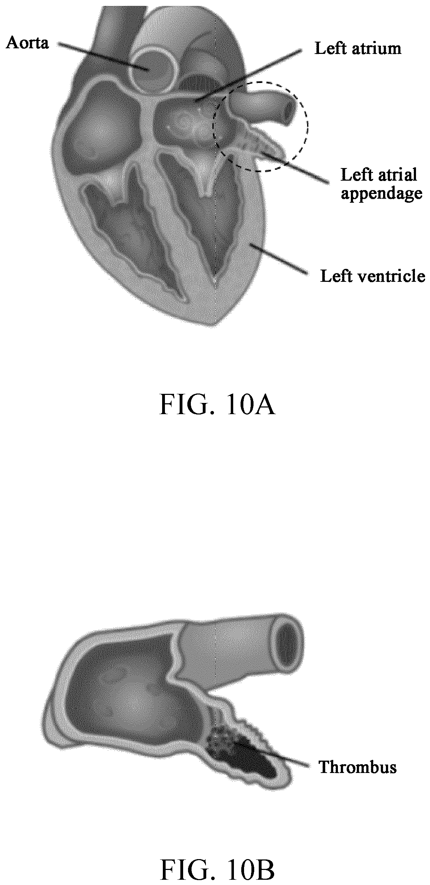

Herein, the technical effects achieved by the present invention are described by using a left atrial appendage as an example. Referring to FIG. 10A and FIG. 10B, since the left atrial appendage has an irregular shape and a limited space, an existing ablation catheter cannot effectively perform ablation. According to the medical ablation catheter provided in the embodiments of the present invention, a strawberry-shaped to-be-ablated region is formed by using a first expandable electrode located on the distal end of the catheter and the plurality of spirally distributed second expandable electrodes, to conform to a shape (in a shape of a strawberry) of an inner lumen of the left atrial appendage. In addition, a surface of each expandable electrode is deformable through the flexibility of the fine metal wire of the expandable electrode, to enhance an adherence capability of the expandable electrode to an inner wall of the left atrial appendage, thereby ensuring the ablation effect.

The medical ablation catheter provided in the embodiments of the present invention may configured to perform the RFA on bilateral superior pulmonary vein ostia, to treat atrial fibrillation. Each of lumen shapes of the superior pulmonary vein ostia varies greatly, so the two-stage elastic structure provided in the embodiments of the present invention needs to be used to improve an adherence capability. Certainly, a person skilled in the art may understand that the medical ablation catheter provided in the embodiments of the present invention is not limited to the above shape of the to-be-ablated region, and may be further configured to perform ablation on another lumen.

It should be noted that the technical features of the above embodiments may be combined in different manners. To make the description concise, not all possible combinations of the technical features in the above embodiments are described. However, the combinations of these technical features shall be considered as falling within the scope recorded in this specification provided that no conflict exists.

Compared with the prior art, according to the medical ablation catheter provided in the embodiments of the present invention, stretching length of the conductive portion may be adjusted by stretching the proximal end of the adjustment portion, and a rotation angle of the conductive portion is adjusted by rotating the proximal end of the adjustment portion, thereby jointly adjusting an operating form of the conductive portion. During specific use, the conductive portion may be adjusted to different shapes such as a circular ring, a spiral, and a straight line as required. When the conductive portion is in the shape of the spiral, magnitudes of a pitch and an outer diameter may be adjusted by stretching or rotating the adjustment portion. Therefore, a plurality of ablation manners can be achieved by using the medical ablation catheter, and different operating forms may be changed as required, thereby improving applicability and convenience in use of the medical ablation catheter.

The medical ablation catheter, the ablation system, and the ablation method provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. A medical ablation catheter, comprising:
a first catheter, located on a distal end of the medical ablation catheter;
a second catheter, comprising an inner catheter and an outer catheter, wherein the outer catheter is sleeved on an outer side of the inner catheter, and is slidable relative to the inner catheter;
a conductive portion, configured to connect to the first catheter and the inner catheter, wherein a distal end of the conductive portion is fixed in the first catheter, and a proximal end of the conductive portion is fixed on the inner catheter; and
an adjustment portion, wherein a distal end of the adjustment portion is fixed in the first catheter, and a proximal end of the adjustment portion is arranged to movably extend through the inner catheter, wherein
the conductive portion may be switchable between a linear state and an expanded state, and ablation is performed when the conductive portion is in the expanded state.

2. The medical ablation catheter according to claim 1, further comprising a first expandable electrode, wherein
the first expandable electrode is located on a distal end of the first catheter, and the first expandable electrode is made of a preformed metal wire or made by carving a metal tube.

3. The medical ablation catheter according to claim 1, wherein
the conductive portion comprises a plurality of second expandable electrodes configured to perform ablation, and each of the second expandable electrodes is made of a preformed flexible metal wire.

4. The medical ablation catheter according to claim 3, wherein
the conductive portion further comprises a plurality of elastic tubes and a support wire; and
the second expandable electrode is arranged between two adjacent elastic tubes, and two ends of the second expandable electrode each have a tail portion configured to connect to each of the elastic tubes.

5. The medical ablation catheter according to claim 4, wherein
the conductive portion further comprises a preformed support wire; and
the support wire is made of a shape memory alloy, and is arranged to extend through the plurality of elastic tubes and the plurality of second expandable electrodes.

6. The medical ablation catheter according to claim 2 or 3, wherein
the first expandable electrode and/or the second expandable electrode is provided with a pressure sensor; and
the pressure sensor is configured to display an adherence position of the first expandable electrode based on a detected pressure, or the pressure sensor is configured to display adherence positions and/or an adherence quantity of the second expandable electrodes based on the detected pressure.

7. The medical ablation catheter according to claim 5, wherein
the first catheter is provided with a first controllably bendable section, to control a shape of a to-be-ablated region of the first expandable electrode.

8. The medical ablation catheter according to claim 5, wherein
a surface of the first catheter is provided with a plurality of positioning scanning apparatuses, to transmit scanning data to an external device, to form a three-dimensional (3D) image of the to-be-ablated region.

9. The medical ablation catheter according to claim 1, further comprising a cold saline perfusion tube, wherein the cold saline perfusion tube is arranged to extend through the first catheter, a first end of the cold saline perfusion tube is located in the first expandable electrode, and a second end of the cold saline perfusion tube is in communication with a cold saline supply apparatus, to deliver cold saline when a surface temperature of the first expandable electrode reaches a preset value.

10. The medical ablation catheter according to claim 1, wherein
the adjustment portion is arranged to extend through and is movable relative to the inner catheter, and the conductive portion is fixedly connected to the inner catheter and is fixedly connected to the first catheter.

11. The medical ablation catheter according to claim 6, wherein
the metal wire of the second expandable electrode is finer than the metal wire of the first expandable electrode.

12. An ablation method based on the medical ablation catheter according to any of claims 1 to 11, the method comprising the following steps:
delivering the first catheter and the conductive portion by using the second catheter;
pushing the first catheter, so that the first catheter and the conductive portion extend out toward a distal end from the second catheter;
pulling and rotating the adjustment portion, to cause the second expandable electrode of the conductive portion to be adjusted to the expanded state, or to cause the first expandable electrode of the first catheter to be adjusted to the expanded state;
selectively energizing a quantity of electrodes on the conductive portion that need to be energized;
stopping energization;
withdrawing the adjustment portion, to receive the first catheter and the conductive portion within the second catheter; and
withdrawing the second catheter.

13. The ablation method according to claim 12, further comprising the following steps:
pulling and rotating the adjustment portion after the energization is stopped, to cause the conductive portion to be adjusted to the linear state and be withdrawn into the second catheter;
causing the first expandable electrode on the first catheter to be energized; and
stopping the energization, and withdrawing the first catheter into the second catheter.

14. An ablation system, comprising an ablation energy source and the medical ablation catheter according to any of claims 1 to 11, wherein the ablation energy source is configured to provide energy to the ablation catheter.

15. The ablation system according to claim 14, wherein
the ablation energy source comprises at least an energy source for pulsed field ablation (PFA) and an energy source for radio-frequency ablation (RFA).
